# EUROPÄISCHE PATENTANMELDUNG

(11) **EP 1 036 571 A2**
(43) Veröffentlichungstag der Anmeldung: **20.09.2000**
(21) Anmeldenummer: 00105484.0
(22) Anmeldetag: 15.03.2000
(51) Int. Cl.: A61M 39/02, A61M 5/32

(54) **Kanüle für medizinischen Zugangsports**

(30) Priorität: 17.03.1999 DE 19911970
(71) Anmelder: MEDINORM AKTIENGESELLSCHAFT MEDIZINTECHNISCHE PRODUKTE, D-66287 Quierschied (DE)
(72) Erfinder: März, Peter, Dr., 82377 Penzberg (DE)
(74) Vertreter: Schlimme, Wolfram, Dr.-Ing.

(57) **Zusammenfassung**

Eine Kanüle für Ports umfaßt einen hohlen Schaft, eine am ersten, vorderen Ende des Portkanüle ausgebildete Spitze und einen am zweiten, hinteren Ende der Portkanüle vorgesehenen Fluidanschluß, wobei im Bereich des ersten Endes zumindest eine Fluiddurchtrittsöffnung in der Wandung des Schaftes vorgesehen ist. Die Spitze ist im wesentlichen kegelförmig und geschlossen ausgebildet und die zumindest eine Fluiddurchtrittsöffnung ist von einem Längsschlitz in der seitlichen Wandung des Schaftes, dem ersten Ende benachbart, gebildet.

## Beschreibung

Die Erfindung betrifft eine Kanäle für Ports, umfassend einen hohlen Schaft, eine am ersten, vorderen Ende der Portkanüle ausgebildete Spitze und einen am zweiten, hinteren Ende der Portkanüle vorgesehenen Fluidanschluß, wobei im Bereich des ersten Endes zumindest eine Fluiddurchtrittsöffnung in der Wandung des Schaftes vorgesehen ist.

Sogenannte Ports sind künstliche Köperzugänge, die unter die Haut eines Patienten eingesetzt werden und mittels eines Katheters mit einem Gefäß oder Organ verbunden werden. Diese Ports werden beispielsweise als künstliche Venenzugänge eingesetzt, bei denen der Katheter in eine Vene eingeführt ist. Es sind aber auch Ports als künstliche Arterienzugänge, spinale Zugänge oder peridurale Zugänge bekannt. Generell kann mittels eines Ports Flüssigkeit in den Körper eingeführt werden.

Ports bestehen aus einem Gehäuse mit einem inneren Hohlraum, wobei das Gehäuse einen Katheteranschluß und eine Einstichöffnung aufweist. Der Katheteranschluß ist mit einem Venenkatheter verbunden. Die Einstichöffnung ist über eine im Gehäuse eingespannte Membran verschlossen. Die Membran besteht bevorzugt aus einem Polymer, beispielsweise aus Silikon. Auf der der Membran gegenübergelegenen Innenseite des Ports kann eine Metallplatte vorgesehen sein, die ein Durchstechen der Portrückwand beim Einführen einer Portkanüle in den Port verhindert. Die Metallplatte ist bevorzugt in der Portrückwand eingeschlossen. Alternativ kann die Portrückwand aber auch aus geeigneten anderen harten Materialien, wie beispielsweise einem harten Kunststoff, bestehen, solange dadurch das Durchstechen der Portrückwand verhindert ist.

Der Port, beispielsweise ein künstlicher Venenzugang, wird einem Patienten operativ eingesetzt und zum Beispiel im Brustbereich unter der Haut so fixiert, daß die mit der Membran verschlossene Einstichöffnung zur Körperaußenseite gerichtet und der Venenkatheter in eine Vene eingeführt ist.

Muß bei einem mit diesem Port versehenen Patienten ein Zugang zur Vene hergestellt werden, beispielsweise zur Blutentnahme oder zum Setzen einer Infusion, so wird eine Portkanüle von außen durch die Haut des Patienten und durch die Membran des Ports in den Hohlraum des Ports eingeführt, wodurch eine Fluidverbindung vom Fluidanschluß der Portkanüle durch den Schaft der Portkanüle, durch den Hohlraum des Ports und durch den Katheter in die Vene geschaffen ist.

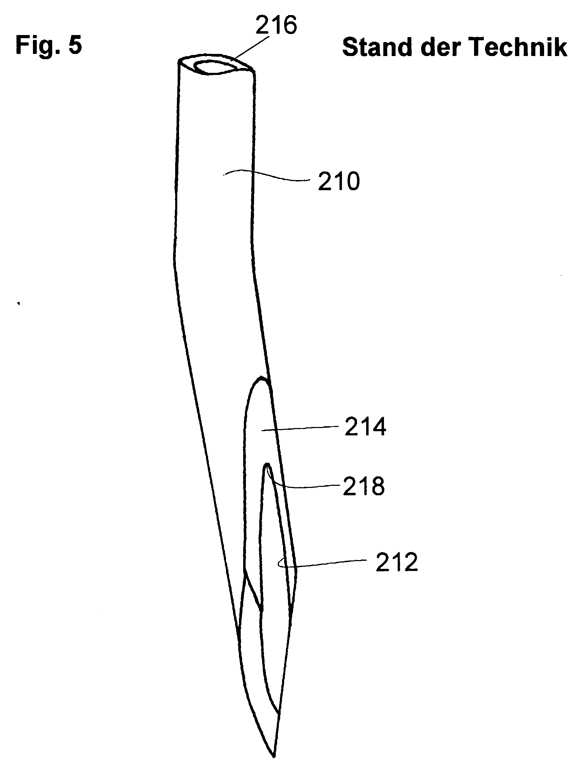

Eine bekannte Portkanüle, eine sogenannte "Huber-Kanüle" ist in Fig. 5 dargestellt. Der Schaft 210 dieser bekannten Portkanüle ist an seinem vorderen Spitzenende in einem spitzen Winkel zur Schaftlängsachse mehrfach angeschliffen, wodurch ein im wesentlichen ovales Schliffauge 212 entsteht, durch welches sich die Kanüle an ihrer Spitze öffnet. Die hintere Verschneidungsfläche 214 des Schliffauges 212 bildet mit der Innenseite der Wandung 216 des Schaftes 210 eine scharfe Kante 218, die beim Durchstechen der zum Beispiel aus Silikon bestehenden Membran hobelartig einen Span aus der Silikonmembran herausschneidet. Hierdurch besteht die Gefahr, daß der herausgeschnittene Silikonspan in den Hohlraum des Ports eingeführt und dort abgelagert wird, so daß er zu einem Verstopfen des Katheters führen oder sogar in die Blutbahn des Patienten gelangen kann. Zudem führt das Abtragen von Spänen aus der Silikonmembran des Ports im Laufe der Zeit zu Undichtigkeiten der Silikonmembran, so daß der Port verhältnismäßig häufig ausgetauscht werden muß, was für den Patienten jedesmal einen operativen Eingriff bedeutet.

Ein weiterer Nachteil der herkömmlichen "Huber-Kanüle" liegt darin, daß die sehr filigrane Spitze beim Auftreffen auf die harte Portrückwand hakenartig verbogen wird, so daß sie beim Herausziehen aus dem Port eine zusätzliche Beschädigung der Silikonmembran bewirkt.

Es ist weiterhin eine Portkanüle bekannt, die an ihrer Spitze einen löffelartigen Schliff aufweist, so daß die hintere Schliffkante bezüglich der Spitze radial einwärts gerichtet ist. Zusätzlich ist bei dieser bekannten Portkanüle der hintere Abschnitt des Schlifffauges durch Kugelstrahlen mit Glasperlen entgratet und abgerundet, so daß hier der Hobeleffekt reduziert ist. Bei dieser bekannten Portkanüle besteht aber ebenfalls die Gefahr, daß die Spitze beim Auftreffen auf die Metallplatte an der Rückwand des Ports verformt wird, so daß beim Herausziehen dieser Portkanüle aus dem Fort ebenfalls eine Beschädigung der Silikonmembran auftreten kann, die dauerhaft zu einer Undichtigkeit der Membran und damit zur Notwendigkeit eines Portwechsels führt.

Außerdem besteht auch bei dieser Portkanüle grundsätzlich die Gefahr, daß sich das durchstochene Membranmaterial während des Hineinstechens der Portkanüle in die Membran aufgrund seiner Elastizität in das verhältnismäßig große Schliffauge hineindrückt und so in Kontakt mit der hinteren Kante des Schliffauges gerät, so daß hier trotz der Entgratung des hinteren Schliffauges grundsätzlich die Möglichkeit einer hobelartigen Abscherung von Membranmaterial besteht.

Aus der DE 80 14 762.2 U1 ist ein Spinalkanüle bekannt, bei der die Spitze in etwa konisch ausgebildet und geschlossen ist und bei der sich eine Schlifföffnung seitlich hinter der Spitze befindet. Diese bekannte Kanüle, die für die Lumbalpunktion vorgesehen ist, besitzt zwar eine konische Spitze, die das zu durchstechende Material nicht zerschneidet sondern verdrängt, schließt aber wegen der seitlichen Schlifföffnung nicht aus, daß das hintere Ende der Schlifföffnung hobelartig wirkt und Späne vom durchstochenen Material abhobelt.

Aufgabe der vorliegenden Erfindung ist es, eine gattungsgemäße Portkanüle so auszugestalten, daß ein Materialabtrag beim Durchstechen der Membran eines Ports verhindert wird und daß auf diese Weise vermieden wird, daß Membranmaterial in den Porthohlraum und damit in den Körper des Patienten gelangt. Weiterhin soll die Lebensdauer der Portmembran und damit des Ports verlängert werden, so daß die Intervalle zwischen zwei Operationen zum Austausch des Ports wesentlich verlängert werden können.

Diese Aufgabe wird gemäß Patentanspruch 1 dadurch gelöst, daß die Spitze im wesentlichen kegelförmig und geschlossen ausgebildet ist und daß die zumindest eine Fluiddurchtrittsöffnung von einem Längsschlitz in der seitlichen Wandung des Schaftes, dem ersten Ende benachbart, gebildet ist.

Durch das Vorsehen der kegelförmigen und geschlossenen Spitze wird zunächst erreicht, daß das durchstochene Gewebe der Haut und auch das durchstochene Material der Silikonmembran nicht zerschnitten sondern nur verdrängt wird. Weiterhin wird durch das Vorsehen des seitlichen Längsschlitzes vermieden, daß scharfe, klingenartige Begrenzungskanten der Öffnung entstehen, wie sie bei den Schliffaugen des Standes der Technik vorhanden sind. Hierdurch wird die Gefahr, daß beim Einstechen der Portkanüle in den Port Silikonspäne aus der Membran herausgeschält werden, vermieden.

Vorzugsweise erstreckt sich der Längsschlitz parallel zur Längsachse des Schaftes.

In einer bevorzugten Ausführungsform ist eine Mehrzahl von Längsschlitzen, vorzugsweise in gleichem Umfangsabstand voneinander, in der Wandung des Schaftes vorgesehen. Durch das Vorsehen mehrerer Längsschlitze kann bei gleichem Gesamtaustrittsquerschnitt die Breite der Schlitze verringert werden, so daß hierdurch die Gefahr, daß das Silikonmaterial der Membran während des Durchstechens in den Schlitz eindringt und dabei beschädigt wird, wesentlich herabgesetzt ist. Zudem wird durch die Mehrzahl von Längsschlitzen ein Spüleffekt innerhalb des Hohlraums des Ports erzielt, der dafür sorgt, daß eventuell im Hohlraum vorhandene Blutreste oder Medikamentenreste aus dem Hohlraum durch den Katheter in die Vene gespült werden. Hierdurch wird vermieden, daß sich im Laufe der Zeit im Hohlraum des Ports Partikel ansammeln, die agglomerieren können und die dann zu einer Verstopfung des Katheters führen können.

Weiter vorzugsweise durchdringt der Längsschlitz die Wandung des Schaftes im wesentlichen radial, wobei die beiden Längsseitenflächen des Längsschlitzes einander zugewandt und vorzugsweise parallel zueinander gelegen sind.

In einer anderen bevorzugten Ausführungsform durchdringt der Längsschlitz die Wandung des Schaftes derart, daß die einander zugewandten Längsseitenflächen des Längsschlitzes in einem spitzen Winkel zur Radialrichtung stehen und vorzugsweise parallel zueinander gelegen sind. Diese Ausgestaltungsform verleiht der aus der Portkanüle in den Hohlraum des Ports austretenden Flüssigkeit eine Strömungsrichtung mit einer Umfangskomponente, so daß die Strömung der aus der Portkanüle austretenden Flüssigkeit innerhalb des Hohlraums des Ports um die Portkanüle herum rotiert, wodurch der Spüleffekt im Hohlraum des Ports besonders wirksam ist.

In einer weiteren bevorzugten Ausgestaltungsform ist die Portkanüle an ihrem vorderen Ende im Bereich der Spitze mit zumindest einer Schliffkante versehen. Hierdurch wird das Eindringen der Portkanüle in die Haut erleichtert, da diese von den Schliffkanten geringfügig angeritzt wird.

Die Erfindung wird nachfolgend anhand eines Beispiels unter Bezugnahme auf die Zeichnung näher erläutert; in dieser zeigt:
- Fig. 1: eine erfindungsgemäße Portkanüle, teilweise im Längsquerschnitt;
- Fig. 2: einen Horizontalquerschnitt durch eine erfindungsgemäße Portkanüle entlang der Linie II-II in Fig. 1;
- Fig. 3: einen Horizontalquerschnitt durch eine alternative Portkanüle, ähnlich Fig. 2;
- Fig. 4: eine perspektivische Darstellung der erfindungsgemäßen Portkanüle mit angedeutetem Fluidaustritt und
- Fig. 5: eine "Huber-Kanüle" des Standes der Technik.

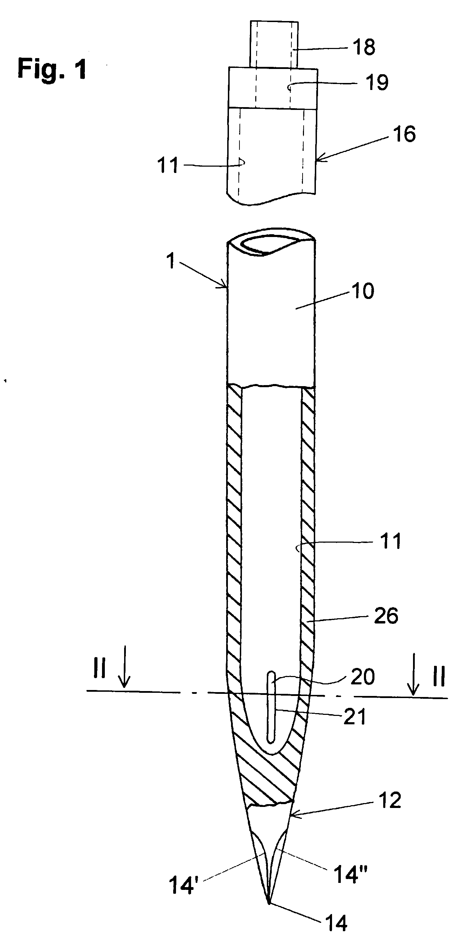

In Fig. 1 ist eine Portkanüle gemäß der Erfindung (in Längsrichtung unterbrochen) dargestellt. Die Portkanüle 1 ist an ihrem vorderen (in Fig. 1 unteren) Ende 12 mit einer im wesentlichen kegelförmigen Spitze 14 versehen. Im Bereich der kegelförmigen Spitze 14 sind zwei Schliffkanten 14', 14'' dargestellt, die das Eindringen der Portkanüle in die Haut erleichtern. Die Schliffkanten sind verhältnismäßig kurz und erstrecken sich von dem Spitzenende der Spitze 14 über etwa ein Drittel der kegelförmig ausgebildeten Spitze 14 am vorderen Ende 12 in Längsrichtung der Portkanüle 1.

Am hinteren (in Fig. 1 oberen) Ende 16 ist ein Fluidanschluß 18 angebracht, der den Anschluß einer Infusion oder einer Spritze ermöglicht, wozu als Verbindung zwischen dem Fluidanschluß 18 und der Infusion beziehungsweise der Spritze ein nicht gezeigter Ansatzschlauch am Fluidanschluß 18 auf bekannte Weise befestigt sein kann.

Zwischen dem vorderen Ende 12 und dem hinteren Ende 16 ist die Portkanüle 1 als hohler Schaft 10 ausgebildet. Das hintere Ende 16 und der hohle Schaft 10 weisen eine sich zum hinteren Ende der Portkanüle 1 öffnende axiale Sacklochbohrung 11 auf, die sich bis in das vordere Ende 12 erstreckt. Auch der Fluidanschluß 18 ist mit einer Axialbohrung 19 versehen, die in Fluidverbindung mit der axialen Sacklochbohrung 11 steht.

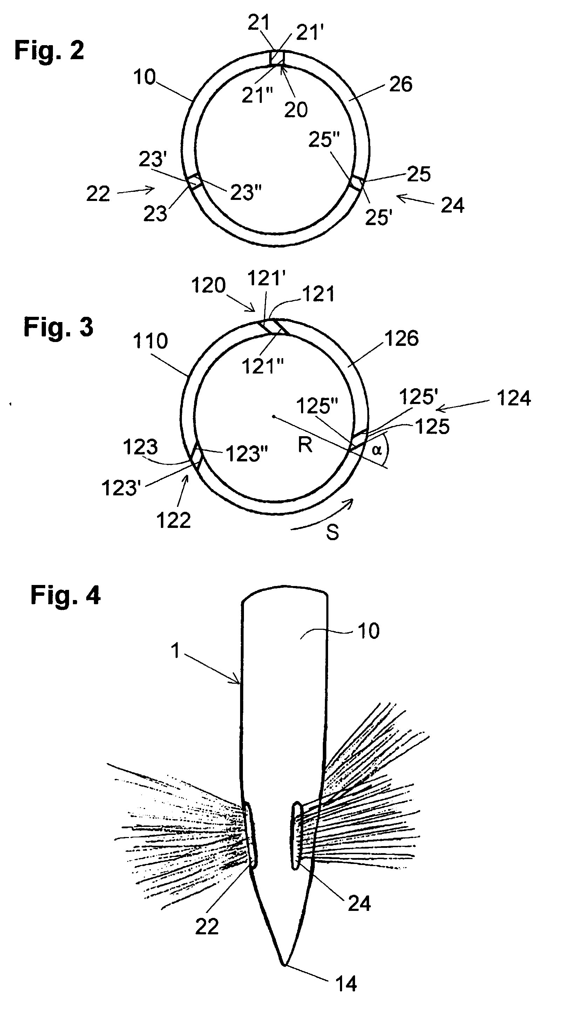

Dem ersten Ende 12 benachbart oder im Übergangsbereich zwischen dem ersten Ende 12 und dem Schaft 10 sind in gleichem Winkelabstand über den Umfang der Portkanüle 1 verteilt drei Fluiddurchtrittsöffnungen 20, 22, 24 (siehe Fig. 2) vorgesehen. Die Fluiddurchtrittsöffnungen 20, 22, 24 sind von Längsschlitzen 21, 23, 25 gebildet, die die seitliche Wandung 26 des Schaftes 10 beziehungsweise des Übergangsbereichs zwischen dem ersten Ende 12 und dem Schaft 10 im wesentlichen radial durchdringen. Die Längsschlitze 21, 23, 25 erstrecken sich in ihrer Längsausdehnung parallel zur Längsachse der Portkanüle 1, wobei die Längsausdehnung der Längsschlitze 21, 23, 25 ein Vielfaches von deren Breite beträgt.

Wie in Fig. 2 zu sehen ist, verlaufen die Längsseitenflächen 21', 21''; 23', 23''; 25', 25'' eines jedes Längsschlitzes 21, 23, 25 jeweils (bezogen auf den jeweiligen Längsschlitz) parallel zueinander und parallel zu einer durch die Längsmittelachse des betreffenden Längsschlitzes und durch die Längsachse des Schaftes 10 verlaufenden Ebene. Vorzugsweise sind dabei die radial außenliegenden Kanten eines jeden Längsschlitzes 21, 23, 25 abgerundet oder angefast.

In einer alternativen Ausführungsform gemäß Fig. 3 verlaufen die beiden Längsseitenflächen 121', 121''; 123' 123'; 125', 125'' eines jeden Längsschlitzes 121, 123, 125 (bezogen auf den jeweiligen Längsschlitz) parallel zueinander, aber in einem spitzen Winkel α zur Radialrichtung R, wobei der Winkel α größer als 0° und kleiner als 90° ist. Durch diese Ausgestaltung der Längsschlitze erhält die aus der sacklochartigen Axialbohrung 11 durch die Längsschlitze 121, 123, 125 austretenden Flüssigkeitsströmung einen Richtungsvektor, der die Flüssigkeit außerhalb der Portkanüle (das heißt im Hohlraum eines Ports) um die Portkanüle herum in Richtung des Pfeiles S rotieren läßt. Diese Rotation sorgt für einen besonders wirksamen Spüleffekt innerhalb des Porthohlraums.

Fig. 4 zeigt die Kanüle der Fig. 1 im Einsatz, wobei eine durch den nicht dargestellten Fluidanschluß in die axiale Sacklockbohrung eingeleitete Flüssigkeit aus den Fluiddurchtrittsöffnungen 20 (auf der Rückseite), 22 und 24 im wesentlichen radial und fächerartig austritt.

Die Erfindung ist nicht auf das obige Ausführungsbeispiel beschränkt, das lediglich der allgemeinen Erläuterung des Kerngedankens der Erfindung dient. Im Rahmen des Schutzumfangs kann die erfindungsgemäße Vorrichtung vielmehr auch andere als die oben beschriebenen Ausgestaltungsformen annehmen. Die Vorrichtung kann hierbei insbesondere Merkmale aufweisen, die eine Kombination aus den jeweiligen Einzelmerkmalen der Ansprüche darstellen.

Bezugszeichen in den Ansprüchen, der Beschreibung und den Zeichnungen dienen lediglich dem besseren Verständnis der Erfindung und sollen den Schutzumfang nicht einschränken.

## Patentansprüche

1. Kanüle für Ports umfassend
- einen hohlen Schaft (10; 110),
- eine am ersten, vorderen Ende (12) der Portkanüle (1) ausgebildete Spitze (14) und
- einen am zweiten, hinteren Ende (16) der Portkanüle (1) vorgesehenen Fluidanschluß (18),
- wobei im Bereich des ersten Endes (12) zumindest eine Fluiddurchtrittsöffnung (20; 22; 24; 120; 122; 124) in der Wandung (26) des Schaftes (10; 110) vorgesehen ist,
dadurch **gekennzeichnet,**
- daß die Spitze (14) im wesentlichen kegelförmig und geschlossen ausgebildet ist und
- daß die zumindest eine Fluiddurchtrittsöffnung (20; 22; 24; 120; 122; 124) von einem Längsschlitz (21; 23; 25; 121; 123; 125) in der seitlichen Wandung (26; 126) des Schaftes (10; 110), dem ersten Ende (12) benachbart, gebildet ist.

2. Kanüle für Ports nach Anspruch 1,
dadurch **gekennzeichnet,**
daß sich der Längsschlitz (21; 23; 25; 121; 123; 125) parallel zur Längsachse des Schaftes (10) erstreckt.

3. Kanüle für Ports nach Anspruch 1 oder 2,
dadurch **gekennzeichnet,**
daß eine Mehrzahl von Längsschlitzen (21, 23, 25; 121, 123, 125), vorzugsweise in gleichem Umfangsabstand voneinander, in der Wandung (26; 126) des Schaftes (10; 110) vorgesehen ist.

4. Kanüle für Ports nach Anspruch 1, 2 oder 3,
dadurch **gekennzeichnet,**
daß der Längsschlitz (21; 23; 25) die Wandung (26) des Schaftes (10) im wesentlichen radial durchdringt, wobei die beiden Längsseitenflächen (21', 21''; 23', 23''; 25', 25'') des Längsschlitzes (21; 23; 25) einander zugewandt sind.

5. Kanüle für Ports nach Anspruch 1, 2 oder 3,
dadurch **gekennzeichnet,**
daß der Längsschlitz (121; 123; 125) die Wandung (126) des Schaftes (110) derart durchdringt, daß die einander zugewandten Längsseitenflächen (121', 121''; 123', 123''; 125', 125'') des Längsschlitzes (121; 123; 125), in einem spitzen Winkel (α) zur Radialrichtung (R) stehen.

6. Kanüle für Ports nach Anspruch 4 oder 5,
dadurch **gekennzeichnet**,
daß die beiden Längsseitenflächen (21', 21''; 23', 23''; 25', 25''; 121', 121''; 123', 123''; 125', 125'') des Längsschlitzes (21; 23; 25; 121; 123; 125) parallel zueinander gelegen sind.

7. Kanüle für Ports nach einem der vorhergehenden Ansprüche,
dadurch gekennzeichnet,
daß die Portkanüle (1) an ihrem vorderen Ende (12) im Bereich der Spitze (14) mit zumindest einer Schliffkante (14', 14'') versehen ist.
